# EUROPÄISCHE PATENTSCHRIFT

(11) **EP 4 311 507 B1**
(45) Veröffentlichungstag und Bekanntmachung des Hinweises auf die Patenterteilung: **26.02.2025**
(21) Anmeldenummer: 23184110.7
(22) Anmeldetag: 07.07.2023
(51) Int. Cl.: A61B 17/15

(54) **CHIRURGISCHES INSTRUMENT**
SURGICAL INSTRUMENT
INSTRUMENT CHIRURGICAL

(30) Priorität: 25.07.2022 DE 102022207578
(43) Veröffentlichungstag der Anmeldung: 31.01.2024
(73) Patentinhaber: Aesculap AG, 78532 Tuttlingen (DE)
(72) Erfinder: FIRMBACH, Franz-Peter, 78576 Emmingen-Liptingen (DE); ANHORM, Svenja, 72535 Heroldstatt (DE); NONNENMANN, Martin, 78573 Wurmlingen (DE)
(74) Vertreter: Patentanwälte Ruff, Wilhelm, Beier, Dauster & Partner mbB

(56) Entgegenhaltungen:
- AU-A1- 2010 283 577
- US-A1- 2015 045 801
- US-A1- 2021 000 484

## Beschreibung

Die Erfindung betrifft ein chirurgisches Instrument zur Verwendung bei einer Kniegelenkersatzoperation.

Die Verwendung von orthopädischen Prothesen als künstlicher Ersatz für beschädigte oder abgenutzte natürliche Knochenstrukturen ist gängige medizinische Praxis. Insbesondere Hüft- oder Kniegelenkersatzoperationen gehören mittlerweile zum Standardrepertoire der chirurgischen Orthopädie.

Bei einer Kniegelenkersatzoperation (englisch: Total Knee Arthroplasty (TKA)) werden abgenutzte oder anderweitig durch Krankheit oder einen Unfall beeinträchtigte Gelenkflächen des Femurs und/oder der Tibia durch eine Kniegelenkprothese ersetzt. Solche Kniegelenkprothesen umfassen üblicherweise eine Femurkomponente, welche am distalen Ende des Femurs implantiert wird, und eine Tibiakomponente, welche am proximalen Ende der Tibia implantiert wird. Um eine einwandfreie Funktion des künstlichen Gelenkersatzes zu gewährleisten, müssen die besagten Komponenten hinsichtlich ihrer Lage und Orientierung in Bezug auf die Anatomie des Patienten und dessen Körperachsen in definierter Weise möglichst präzise positioniert werden. Andernfalls ist mit einem für den Patienten nicht zufriedenstellenden Ergebnis zu rechnen. Hinsichtlich der Positionierung der Komponenten existieren unterschiedliche chirurgische Ansätze.

Ein als Mechanical Alignment bekannter und bislang vorwiegend verwendeter Ansatz sieht vor, dass die Position und Ausrichtung der künstlichen Gelenkachsen der Kniegelenkprothese mechanisch ideal und insoweit ohne Berücksichtigung etwaiger orthopädischer Fehlstellungen des Patienten festgelegt werden. Hierbei dient oftmals die Längsachse der Tibia als Referenzachse für die Ausrichtung und Positionierung. Klinische Studien haben gezeigt, dass der Mechanical Alignment-Ansatz zu einer als unnatürlich empfundenen Funktion des künstlichen Kniegelenks führen kann.

Als ein weiterer Ansatz ist das sogenannte Kinematic Alignment (nachfolgend auch als KA abgekürzt) bekannt. Bei dieser Vorgehensweise werden die Femurkomponente und die Tibiakomponente unter Berücksichtigung etwaiger orthopädischer Fehlstellungen des Patienten positioniert. Ziel hierbei ist es, die natürliche und unter Umständen mit Fehlstellungen behaftete Gelenkausrichtung des Patienten wiederherzustellen. Klinische Studien haben gezeigt, dass der Kinematic Alignment-Ansatz oftmals mit einer verbesserten Patientenzufriedenheit einhergeht. Insbesondere die Funktion des künstlichen Kniegelenks wird seitens der Patienten als eher natürlich empfunden.

Mit dem Bestreben, die Patientenzufriedenheit weiter zu verbessern, geht ein grundsätzlicher Bedarf nach möglichst präzisen, einfach verwendbaren und kosteneffizienten chirurgischen Instrumenten zur Umsetzung des KA einher.

Die vorliegende Erfindung befasst sich mit einem solchen chirurgischen Instrument, im Speziellen mit einem chirurgischen Instrument zur Positionierung eines Tibiaschnittblocks an der proximalen Tibia. Solche Instrumente werden auch als Übertragungswerkzeug oder tibia transfer tool bezeichnet und erlauben eine Übertragung (transfer) einer femurseitig bereits festgelegten Varus/Valgus-Ausrichtung (nachfolgend V/V-Ausrichtung) auf die Tibia. Aus dem Stand der Technik sind unterschiedliche derartige Instrumente bekannt.

Beispielsweise ist aus der GB 2558543 A ein Instrument mit einem Verlängerungsarm (extension arm) bekannt. Der Verlängerungsarm kann einends an einer femoralen Referenzkomponente befestigt werden. Die Referenzkomponente definiert insbesondere die V/V-Ausrichtung. Die V/V-Ausrichtung ist mittels des Verlängerungsarms auf die proximale Tibia übertragbar. Die Länge des Verlängerungsarms definiert eine proximodistale Position des Tibiaschnittblocks und damit des proximalen Tibiaschnitts (nachfolgend Tibiaschnitthöhe). Der Transfer erfolgt unter Extension, d.h. bei gestrecktem Bein.

Ein weiteres Instrument ist aus der US 2019/0336141 A1 bekannt und einends an einem 4-1-Femurschnittblock und andernends an einem Tibiaschnittblock befestigbar. Zur Einstellung der Tibiaschnitthöhe weist das bekannte Instrument eine Art Linearführung auf. Der Transfer erfolgt unter Flexion, d.h. bei gebeugtem Bein.

Aus der US 10,709,458 B1 sind weitere transfer tools bekannt, die einen Transfer in Extension oder Flexion erlauben.

Das Dokument US 2015/045801 A1 offenbart ein weiteres chirurgisches Instrument zur Verwendung bei einer Kniegelenkersatzoperation.

Aufgabe der Erfindung ist es, ein chirurgisches Instrument bereitzustellen, das Vorteile gegenüber dem Stand der Technik bietet und insbesondere eine verbesserte Positionierung des Tibiaschnittblocks ausgehend von einer femurseitig fixierten Referenzkomponente ermöglicht.

Diese Aufgabe wird durch das Bereitstellen eines chirurgischen Instruments mit den Merkmalen des Anspruchs 1 gelöst.

Das erfindungsgemäße chirurgische Instrument weist auf: eine erste Befestigungseinrichtung, welche zur lösbaren Befestigung an einer an einem distalen Femur angebrachten Referenzkomponente eingerichtet ist, eine zweite Befestigungseinrichtung, welche distal von der ersten Befestigungseinrichtung beabstandet und zur lösbaren Befestigung an einem Tibiaschnittblock zur Schnittführung an einer proximalen Tibia eingerichtet ist, eine Kreisbogenführung, welche einends mit der ersten Befestigungseinrichtung und andernends mit der zweiten Befestigungseinrichtung verbunden ist und mittels derer die zweite Befestigungseinrichtung relativ zu der ersten Befestigungseinrichtung in einer sagittalen Führungsebene entlang einer kreisbogenförmig längserstreckten Führungsbahn geführt linearbeweglich ist, eine erste Linearführung, mittels derer die Kreisbogenführung einends mit der ersten Befestigungseinrichtung verbunden und relativ zu derselben in der Führungsebene entlang einer anteroposterior längserstreckten ersten Führungsbahn geführt linearbeweglich ist, und eine zweite Linearführung, mittels derer die zweite Befestigungseinrichtung andernends mit der Kreisbogenführung verbunden und relativ zu derselben in der Führungsebene entlang einer anteroposterior längserstreckten zweiten Führungsbahn geführt linearbeweglich ist. Das erfindungsgemäße chirurgische Instrument erlaubt eine verbesserte Positionierung des Tibiaschnittblocks. Die verbesserte Positionierung wird durch ein Zusammenwirken der Kreisbogenführung, der ersten Linearführung und der zweiten Linearführung erreicht. In der Verwendung des chirurgischen Instruments ist die erste Befestigungseinrichtung lösbar an der femurseitig angebrachten Referenzkomponente befestigt und der Tibiaschnittblock ist lösbar an der zweiten Befestigungseinrichtung befestigt. Die Referenzkomponente ist beispielsweise ein distaler Femurschnittblock zur distalen Schnittführung an dem Femur oder dergleichen. Weder die Referenzkomponente noch der Tibiaschnittblock sind Bestandteile des erfindungsgemäßen chirurgischen Instruments. Die Kreisbogenführung erlaubt eine geführte kreisbogenförmige Bewegung der zweiten Befestigungseinrichtung relativ zu der ersten Befestigungseinrichtung. In der Verwendung des chirurgischen Instruments erlaubt die Kreisbogenführung folglich eine dementsprechend geführte Relativbewegung des Tibiaschnittblocks gegenüber der femurseitigen Referenzkomponente. Hierdurch kann die Neigung des Tibiaschnittblocks in der Führungsebene eingestellt werden. Die Führungsebene ist sagittal ausgerichtet und folglich anteroposterior sowie proximodistal erstreckt. Die besagte Neigung wird auch als posteriorer oder anteriorer Slope bezeichnet. Die Einstellbarkeit der Neigung des Tibiaschnittblocks (nachfolgend kurz Slope) erlaubt zum einen eine Anpassung an präoperativ festgelegte Parameter. Zum anderen kann eine tatsächlich vorherrschende Flexions- oder Extensionsstellung des Beins durch die Beweglichkeit der Kreisbogenführung berücksichtigt und ausgeglichen werden. Die Kreisbogenführung ist relativ zu der ersten Befestigungseinrichtung entlang der ersten Führungsbahn beweglich. Hierdurch kann eine anteroposteriore Position der kreisbogenförmigen Führungsbahn in Relation zu der ersten Befestigungseinrichtung - und damit der Referenzkomponente - verändert und an die Anatomie des Patienten angepasst werden. Im Idealfall wird die Position der kreisbogenförmigen Führungsbahn derart angepasst, dass deren Kreismittelpunkt auf der mechanischen Tibiaachse zum Liegen kommt. Hierdurch ist gewährleistet, dass eine Einstellung des Slope nicht gleichzeitig zu einer ungewollten Veränderung einer proximodistalen Position des Tibiaschnittblocks und damit der Tibiaschnitthöhe führt. Die zweite Befestigungseinrichtung ist relativ zu der Kreisbogenführung entlang der zweiten Führungsbahn beweglich. Hierdurch ist gewährleistet, dass eine anteroposteriore Position der zweiten Befestigungseinrichtung - und damit die des Tibiaschnittblocks - an die Anatomie des Patienten, insbesondere die Größe der Tibia, anpassbar ist, ohne dass eine ungewollte Veränderung der anteroposterioren Position der kreisbogenförmigen Führungsbahn stattfindet.

Die in dieser Beschreibung verwendeten Lage- und Richtungsbezeichnungen beziehen sich auf den Körper eines Patienten, insbesondere dessen Femur, und sind insoweit gemäß ihrer üblichen anatomischen Bedeutung zu verstehen. Folglich bedeutet "anterior" vordere(r) oder vorne liegend, "posterior" hintere(r) oder hinten liegend, "medial" innere(r) oder innen liegend, "lateral" äußere(r) oder außen liegend, "proximal" zum Körperzentrum hin und "distal" vom Körperzentrum entfernt. Weiter bedeuten "proximodistal" entlang, vorzugsweise parallel zu, einer proximal-distal ausgerichteten Achse, "anteroposterior" entlang, vorzugsweise parallel zu, einer anterior-posterior ausgerichteten Achse und "mediolateral" entlang, vorzugsweise parallel zu, einer medial-lateral ausgerichteten Achse. Die besagten Achsen sind orthogonal zueinander ausgerichtet und können selbstverständlich in Bezug zu nicht mit der Anatomie des Patienten in Verbindung stehenden X-, Y- und Z-Achsen gesetzt werden. Beispielsweise kann die proximaldistale Achse alternativ als X-Achse bezeichnet werden. Die medial-laterale Achse kann als Y-Achse bezeichnet werden. Die anterior-posteriore Achse kann als Z-Achse bezeichnet werden. Zur verbesserten Veranschaulichung und der Einfachheit der Bezeichnungen wegen werden nachfolgend in erster Linie die besagten anatomischen Lage- und Richtungsbezeichnungen verwendet.

In Ausgestaltung der Erfindung ist ein Ausrichtstab vorhanden und zur Ausrichtung an einer anterioren Kante der Tibia eingerichtet, wobei der Ausrichtstab einends relativ zu der zweiten Befestigungseinrichtung um eine orthogonal zu der Führungsebene orientierte Schwenkachse schwenkbeweglich an der zweiten Befestigungseinrichtung gelagert ist. Der Ausrichtstab erlaubt eine Referenzierung an der besagten anterioren Kante, die auch als Tibiavorderkante bezeichnet werden kann. Der Slope (anteriore oder posteriore Neigung des Tibiaschnitts) wird präoperativ oftmals in Bezug auf die Tibiavorderkante festgelegt. Der Ausrichtstab erlaubt eine einfache Kontrolle des Slope in Bezug auf die Tibiavorderkante. Der Ausrichtstab ist zwischen einem ersten Ende und einem zweiten Ende längserstreckt. Das erste Ende ist schwenkbeweglich an der zweiten Befestigungseinrichtung gelagert. Das zweite Ende ist der zweiten Befestigungseinrichtung distal abgewandt.

In weiterer Ausgestaltung der Erfindung ist eine erste Anzeigerichtung vorhanden und zum Anzeigen einer ersten Winkelstellung der zweiten Befestigungseinrichtung in Bezug auf die kreisbogenförmige Führungsbahn eingerichtet, und/oder eine zweite Anzeigeeinrichtung ist vorhanden und zum Anzeigen einer zweiten Winkelstellung des Ausrichtstabs in Bezug auf die Schwenkachse eingerichtet. Die erste Anzeigeeinrichtung dient einer Anzeige der relativen sagittalen Neigung zwischen der ersten Befestigungseinrichtung und der zweiten Befestigungseinrichtung. In der Verwendung des chirurgischen Instruments - mit femurseitig befestigter erster Befestigungseinrichtung und an der zweiten Befestigungseinrichtung befestigtem Tibiaschnittblock - zeigt die erste Anzeigeeinrichtung den Slope des Tibiaschnittblocks und damit auch des Tibiaschnitts an. Die zweite Anzeigeeinrichtung dient einer Anzeige einer relativen sagittalen Neigung zwischen dem Ausrichtstab und der zweiten Befestigungseinrichtung. In der Verwendung des chirurgischen Instruments kann mittels der zweiten Anzeigeeinrichtung eine Abweichung der Flexion/Extensions-Stellung ermittelt werden. Die ermittelte Abweichung kann im Nachgang über eine entsprechende Einstellung des Slope ausgeglichen werden. Hierfür wird der Slope zunächst auf 0° eingestellt. Dies kann über eine entsprechende Bewegung der zweiten Befestigungseinrichtung entlang der kreisbogenförmig längserstreckten Führungsbahn geschehen. Hiernach kann der Ausrichtstab parallel zu der anterioren Kante der Tibia ausgerichtet werden. Die sich hierbei ergebende zweite Winkelstellung wird mittels der zweiten Anzeigeeinrichtung angezeigt. Die erste Winkelstellung kann hiernach um den Wert der zweiten Winkelstellung korrigiert werden. Hieraus resultiert ein tatsächlicher Slope von 0°, ungeachtet einer etwaigen Abweichung von der exakten Extension. Im Nachgang kann eine nochmalige Einstellung des Slope auf einen präoperativ festgelegten Wert erfolgen. Vorzugsweise ist die erste Anzeigeeinrichtung eine an der Kreisbogenführung angebrachte Skale. Vorzugsweise ist die zweite Anzeigeeinrichtung eine an dem Ausrichtstab und/oder der zweiten Befestigungseinrichtung angeordnete Skale. Die Skalen weisen vorzugsweise jeweils eine Ablesemarke und Teilstriche zum Anzeigen und/oder Ablesen der jeweiligen Winkelstellung auf. Alternativ ist es denkbar, dass die erste Winkelstellung und/oder die zweite Winkelstellung mittels hierfür geeigneter Sensoren erfasst und digital angezeigt werden. Dementsprechend kann die erste Anzeigeeinrichtung und/oder die zweite Anzeigeeinrichtung auch als Display gestaltet sein.

In weiterer Ausgestaltung der Erfindung weist die Kreisbogenführung wenigstens eine gebogene Führungsstange und eine Führungsaufnahme auf, in welcher die Führungsstange gleitbeweglich geführt aufgenommen ist, wobei die Führungsaufnahme mit der ersten Linearführung und die Führungsstange mit der zweiten Linearführung verbunden ist oder umgekehrt. Die Führungsstange ist im Wesentlichen proximodistal längserstreckt und - in Bezug auf eine mediolateral gerichtete Blickrichtung - posterior konkav und/oder anterior konvex gebogen. Die Biegung der Führungsstange und/oder der Führungsaufnahme definiert die kreisbogenförmig längserstreckte Führungsbahn. Entsprechendes gilt sinngemäß für die Führungsaufnahme. Um eine ungewollte Rotation um die Längsachse der Führungsstange zu vermeiden, weist die Führungsstange vorzugsweise einen nicht rotationssymmetrischen Querschnitt auf. Entsprechendes gilt sinngemäß für die Führungsaufnahme, in welcher die Führungsstange aufgenommen ist. Bei einer Ausgestaltung ist die Führungsaufnahme mit der ersten Linearführung verbunden und die Führungsstange ist mit der zweiten Linearführung verbunden. Bei einer weiteren Ausgestaltung ist die Führungsstange mit der ersten Linearführung verbunden und die Führungsaufnahme ist mit der zweiten Linearführung verbunden.

In weiterer Ausgestaltung der Erfindung ist die Führungsaufnahme mit der ersten Linearführung verbunden und weist ein Führungselement, insbesondere ein Zylinderelement oder eine Zylinderaufnahme, derselben auf, und/oder die Führungsstange ist mit der zweiten Linearführung verbunden und weist ein Führungselement, insbesondere einen Kulissenstein oder einen Kulissenschlitz, derselben auf. Die Führungsaufnahme ist fest und/oder relativ unbeweglich mit dem besagten Führungselement der ersten Linearführung verbunden. Bei einer Ausgestaltung ist das Führungselement der ersten Linearführung ein Zylinderelement, bei einer weiteren Ausgestaltung eine Zylinderaufnahme, in welcher das Zylinderelement gleitbeweglich entlang der ersten Führungsbahn geführt ist. Die Führungsstange ist fest und/oder relativ unbeweglich mit dem besagten Führungselement der zweiten Linearführung verbunden. Bei einer Ausgestaltung ist das Führungselement der zweiten Linearführung ein Kulissenstein, bei einer weiteren ein Kulissenschlitz, in welcher der Kulissenstein gleitbeweglich entlang der zweiten Führungsbahn geführt ist.

In weiterer Ausgestaltung der Erfindung ist die erste Linearführung eine Zylinderführung und weist wenigstens ein anteroposterior längserstrecktes Zylinderelement und eine Zylinderaufnahme auf, in welcher das Zylinderelement gleitbeweglich geführt aufgenommen ist. Die Längserstreckung des Zylinderelements und/oder der Zylinderaufnahme definiert die erste Führungsbahn. Die Zylinderführung ermöglicht eine gleitbewegliche Relativverlagerung der Kreisbogenführung in Bezug auf die erste Befestigungseinrichtung und damit - in der Verwendung des chirurgischen Instruments - in Bezug auf die Referenzkomponente. Diese Ausgestaltung der ersten Linearführung ist besonders robust und präzise. Um eine ungewollte Rotation um die Längsachse des Zylinderelements und/oder der Zylinderaufnahme zu vermeiden, weist das Zylinderelement vorzugsweise einen nicht rotationssymmetrischen Querschnitt auf. Entsprechendes gilt vorzugsweise sinngemäß für die Zylinderaufnahme. Alternativ können das Zylinderelement und/oder die Zylinderaufnahme einen kreisrunden Querschnitt aufweisen. Um in diesem Fall eine ungewollte Rotation um die Längsachse zu unterbinden, weist die Zylinderführung vorzugsweise zwei mediolateral beabstandet angeordnete Zylinderelemente und Zylinderaufnahmen auf.

In weiterer Ausgestaltung der Erfindung ist das Zylinderelement unbeweglich mit der ersten Befestigungseinrichtung verbunden und die Zylinderaufnahme ist unbeweglich mit der Kreisbogenführung, vorzugsweise deren Führungsaufnahme, verbunden. Hierdurch wird ein weiter vereinfachter Aufbau des chirurgischen Instruments erreicht.

In weiterer Ausgestaltung der Erfindung ist die zweite Linearführung eine Kulissenführung und weist einen Kulissenstein und einen anteroposterior längserstreckten Kulissenschlitz auf, in welchem der Kulissenstein gleitbeweglich geführt aufgenommen ist. Der Kulissenschlitz definiert die zweite Führungsbahn. Da die zweite Linearführung gemeinsam mit der zweiten Befestigungseinrichtung entlang der kreisbogenförmigen Führungsbahn beweglich ist, verändert sich die Orientierung des längserstreckten Kulissenschlitzes naturgemäß dementsprechend. Die Orientierung des Kulissenschlitzes ist unabhängig von der Position der zweiten Befestigungseinrichtung entlang der kreisbogenförmigen Führungsbahn wenigstens im Wesentlichen anteroposterior.

In weiterer Ausgestaltung der Erfindung ist der Kulissenstein unbeweglich mit der Kreisbogenführung, vorzugsweise deren Führungsstange, verbunden, und der Kulissenschlitz ist an der zweiten Befestigungseinrichtung ausgebildet. Hierdurch wird ein weiter vereinfachter Aufbau des chirurgischen Instruments erreicht. Vorzugsweise ist der Kulissenstein an einem der ersten Befestigungseinrichtung abgewandten Ende der Führungsstange angeordnet und/oder ausgebildet. Vorzugsweise ist der Kulissenstein einstückig an das besagte Ende der Führungsstange angeformt.

In weiterer Ausgestaltung der Erfindung weist die erste Befestigungseinrichtung wenigstens ein Steckelement auf, welches zum lösbaren Zusammenstecken mit einer komplementären Referenzgeometrie der Referenzkomponente eingerichtet ist. Hierdurch kann die erste Befestigungseinrichtung auf besonders einfache Weise lösbar an der Referenzkomponente befestigt werden. Bei einer Ausgestaltung ist die Referenzkomponente ein distaler Femurschnittblock, welcher zur Aufnahme und Führung eines Sägeblatts bei der Anbringung eines distalen Femurschnitts eingerichtet ist. Zu diesem Zweck weist der Femurschnittblock wenigstens einen Aufnahmeschlitz für das besagte Sägeblatt auf. Vorzugsweise ist das wenigstens eine Steckelement zum Einstecken in den Führungsschlitz eingerichtet. Der Führungsschlitz bildet bei dieser Ausgestaltung die komplementäre Referenzgeometrie.

In weiterer Ausgestaltung der Erfindung weist die zweite Befestigungseinrichtung einen Grundkörper auf, an welchem ein Befestigungselement, welches zur lösbaren Befestigung an einem hierfür vorgesehenen Befestigungsabschnitt des Tibiaschnittblocks eingerichtet ist, ein Führungselement der zweiten Linearführung, vorzugsweise deren Kulissenschlitz, und der Ausrichtstab angeordnet sind. Hierdurch wird ein weiter vereinfachter Aufbau des chirurgischen Instruments erreicht. Das Befestigungselement ist bei unterschiedlichen Ausgestaltungen unterschiedlich ausgeführt und kann beispielsweise ein Klemm-, Rast-, Steck- und/oder Schnappelement zur lösbaren Befestigung an dem Tibiaschnittblock sein. Bei einer Ausgestaltung ist das Befestigungselement relativ zu dem Grundkörper zwischen einer Befestigungsstellung und einer Freigabestellung verlagerbar. In der Befestigungsstellung sind die zweite Befestigungseinrichtung und der Tibiaschnittblock mittels des Befestigungselements lösbar miteinander verbunden. In der Freigabestellung ist die zweite Befestigungseinrichtung von dem Tibiaschnittblock freigegeben und umgekehrt. Der Ausrichtstab ist um die Schwenkachse relativ schwenkbeweglich an dem Grundkörper gelagert. Sofern eine zweite Anzeigeeinrichtung zum Anzeigen einer zweiten Winkelstellung des Ausrichtstabs in Bezug auf die Schwenkachse gemäß einer der vorhergehenden Ausgestaltungen vorhanden ist, ist dieselbe vorzugsweise als Skale an dem Grundkörper und/oder dem Ausrichtstab angeordnet.

In weiterer Ausgestaltung der Erfindung ist eine Fixiereinrichtung vorhanden und zur Fixierung der Beweglichkeit der Kreisbogenführung eingerichtet. Mittels der Fixiereinrichtung ist die zweite Befestigungseinrichtung entlang der kreisbogenförmigen Führungsbahn relativ zu der ersten Befestigungseinrichtung unbeweglich fixierbar. Hierdurch wird einer ungewollten Verlagerung der zweiten Befestigungseinrichtung entlang der kreisbogenförmigen Führungsbahn entgegengewirkt. In der Verwendung des chirurgischen Instruments wirkt die Fixiereinrichtung einer ungewollten Verstellung des Slope entgegen. Die Fixiereinrichtung wirkt form- und/oder kraftschlüssig zwischen unterschiedlichen Bauteilen und/oder Abschnitten der Kreisbogenführung. Sofern die Kreisbogenführung eine Führungsstange und eine Führungsaufnahme aufweist, sind die besagten Bauteile mittels der Fixiereinrichtung relativ zueinander fixierbar.

Die Erfindung betrifft zudem ein chirurgisches Instrumentensystem mit einem chirurgischen Instrument nach der vorhergehenden Beschreibung, einer Referenzkomponente, die lösbar an der ersten Befestigungseinrichtung des chirurgischen Instruments befestigt ist, und mit einem Tibiaschnittblock, der lösbar an der zweiten Befestigungseinrichtung des chirurgischen Instruments befestigt ist..

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.
- Fig. 1: zeigt eine schematische Seitenansicht einer Ausführungsform eines erfindungsgemäßen chirurgischen Instruments in einer intraoperativen Situation, in welcher das chirurgische Instrument einends an einem distalen Femurschnittblock und andernends an einem proximalen Tibiaschnittblock lösbar befestigt ist,
- Fig. 2: eine schematische Perspektivdarstellung der in Fig. 1 gezeigten intraoperativen Situation,
- Fig. 3: eine weitere schematische Perspektivdarstellung der in den Fig. 1 und 2 gezeigten intraoperativen Situation,
- Fig. 4: eine weitere Darstellung der intraoperativen Situation in einer schematischen Seitenansicht und
- Fig. 5: eine schematische Seitenansicht des chirurgischen Instruments in einer weiteren intraoperativen Situation, in welcher der Tibiaschnittblock mittels des chirurgischen Instruments sagittal geneigt ausgerichtet ist.

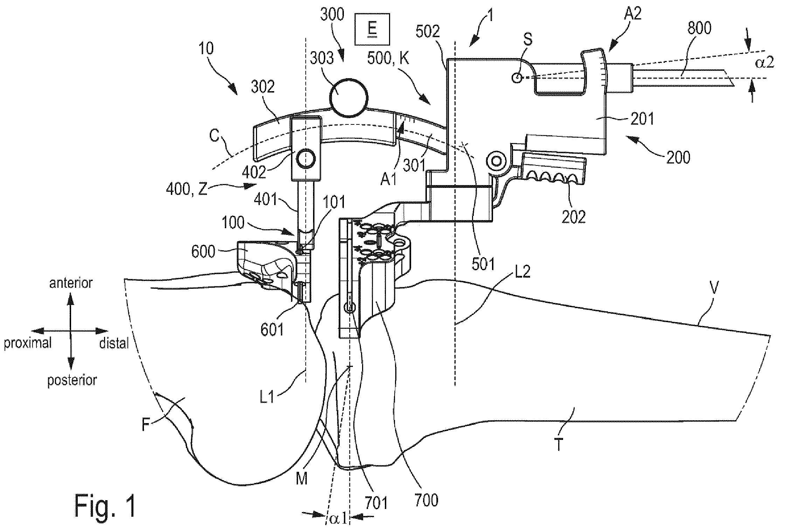

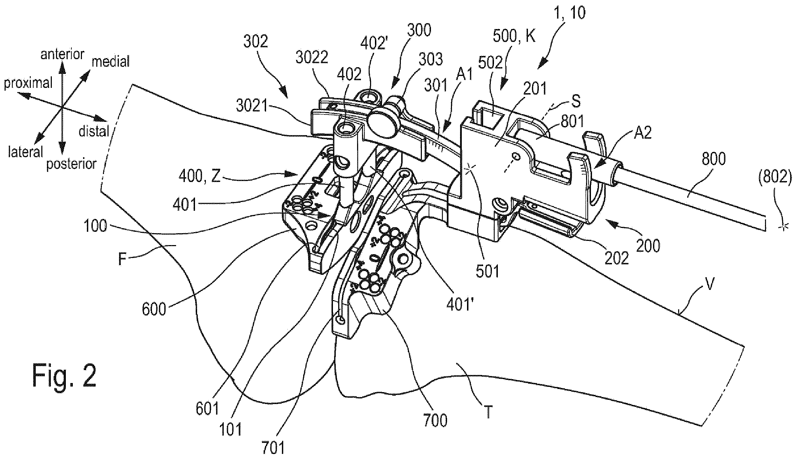

Gemäß den Fig. 1 bis 5 ist ein chirurgisches Instrument 1 zur Verwendung bei einer Kniegelenkersatzoperation vorgesehen. Das chirurgische Instrument 1 kann auch als Übertragungswerkzeug oder tibia transfer tool bezeichnet werden und dient einer referenzierten Positionierung eines Tibiaschnittblocks 700 an einer proximalen Tibia T. Als Referenz für die Position des Tibiaschnittblocks 700 dient ein bereits an einem distalen Femur F positionierter distaler Femurschnittblock 600. Der Femurschnittblock 600 und der Tibiaschnittblock 700 sind jeweils nicht Bestandteil des chirurgischen Instruments 1. Das chirurgische Instrument 1 ist auf noch näher beschriebene Weise lösbar einerseits mit dem Femurschnittblock 600 und andererseits mit dem Tibiaschnittblock 700 verbindbar. Das chirurgische Instrument 1 bildet gemeinsam mit den beiden Schnittblöcken 600, 700 ein chirurgisches Instrumentensystem 10.

Das chirurgische Instrument 1 weist eine erste Befestigungseinrichtung 100, eine zweite Befestigungseinrichtung 200, eine Kreisbogenführung 300, eine erste Linearführung 400 und eine zweite Linearführung 500 auf.

Die erste Befestigungseinrichtung 100 ist zur lösbaren Befestigung an dem distalen Femurschnittblock 600 eingerichtet. Der Femurschnittblock 600 dient insoweit als Referenzkomponente zur Referenzierung der Position und Ausrichtung des Tibiaschnittblocks 700.

Die zweite Befestigungseinrichtung 200 ist distal von der ersten Befestigungseinrichtung 100 beabstandet und zur lösbaren Befestigung an dem Tibiaschnittblock 700 eingerichtet.

Die Kreisbogenführung 300 ist einends mit der ersten Befestigungseinrichtung 100 und andernends mit der zweiten Befestigungseinrichtung 200 verbunden. Die Verbindungen sind jeweils mittelbar oder indirekt. Dabei ist die Kreisbogenführung 300 einends mittels der ersten Linearführung 400 mit der ersten Befestigungseinrichtung 100 verbunden. Andernends ist die Kreisbogenführung 300 mittels der zweiten Linearführung 500 mit der zweiten Befestigungseinrichtung 200 verbunden. Die Kreisbogenführung 300 weist eine kreisbogenförmig längserstreckte Führungsbahn C auf. Die kreisbogenförmige Führungsbahn C (nachfolgend auch Kreisführungsbahn) ist in einer Führungsebene E angeordnet. Die Führungsebene E verläuft proximodistal und anteroposterior. Die Führungsebene E ist eine sagittale Ebene. Die Kreisbogenführung 300 erlaubt eine geführte Relativbeweglichkeit zwischen der zweiten Befestigungseinrichtung 200 und der ersten Befestigungseinrichtung 100. Die zweite Befestigungseinrichtung 200 ist relativ zu der ersten Befestigungseinrichtung 100 entlang der Kreisführungsbahn C geführt linearbeweglich. Dabei führt die zweite Befestigungseinrichtung 200 eine Rotation und/oder Schwenkbewegung um einen Mittelpunkt M der Kreisführungsbahn C aus. Der Mittelpunkt M liegt posterior von der Kreisführungsbahn C beabstandet. Mit anderen Worten ist die Kreisführungsbahn C posterior konkav und anterior konvex gebogen. Der Mittelpunkt M kann auf noch näher beschriebene Weise relativ zu Gelenkachsen des Patienten, im Speziellen zu der Tibiaachse (ohne Bezugszeichen) positioniert werden.

Die erste Linearführung 400 ist einends mit der ersten Befestigungseinrichtung 100 und andernends mit der Kreisbogenführung 300 verbunden. Die erste Linearführung 400 etabliert eine relativbewegliche Verbindung zwischen der ersten Befestigungseinrichtung 100 und der Kreisbogenführung 300. Die erste Linearführung 400 weist eine erste Führungsbahn L1 auf. Die erste Führungsbahn L1 ist anteroposterior längserstreckt und in der Führungsebene E angeordnet und/oder parallel versetzt zu derselben. Die erste Führungsbahn L1 ist gerade längserstreckt.

Die zweite Linearführung 500 ist einends mit der Kreisbogenführung 300 und andernends mit der zweiten Befestigungseinrichtung 200 verbunden. Die zweite Linearführung 500 etabliert eine relativbewegliche Verbindung zwischen der Kreisbogenführung 300 und der zweiten Befestigungseinrichtung 200. Die zweite Linearführung 500 weist eine zweite Führungsbahn L2 auf. Die zweite Führungsbahn L2 ist gerade längserstreckt. Die zweite Führungsbahn L2 ist in der Führungsebene E angeordnet und/oder parallel versetzt zu derselben. In der anhand Fig. 1 gezeigten intraoperativen Situation ist die zweite Führungsbahn L2 parallel zu der ersten Führungsbahn L1 und somit anteroposterior längserstreckt. Die Orientierung der zweiten Führungsbahn L2 ist bei der gezeigten Ausführungsform abhängig von der Position der zweiten Befestigungseinrichtung 200 entlang der Kreisführungsbahn C.

Durch das Zusammenwirken der Kreisbogenführung 300, der ersten Linearführung 400 und der zweiten Linearführung 500 kann der Tibiaschnittblock 700 in einer gegenüber dem Stand der Technik verbesserten Weise ausgehend von dem positionierten Femurschnittblock 600 an der Tibia T positioniert werden. Zum einen kann die sagittale Neigung des Tibiaschnittblocks 700 auf noch näher beschriebene Weise ohne ungewollte Beeinflussung der sogenannten Tibiaschnitthöhe eingestellt werden. Die sagittale Neigung wird auch als Slope bezeichnet. Dabei erfolgt die Positionierung des Tibiaschnittblocks 700 in Extension, d.h. bei gestrecktem Bein und/oder Kniegelenk. In Extension schließen der Femur F und die Tibia T einen Winkel von 180° ein. Mittels des chirurgischen Instruments 1 können Abweichungen von der exakten Extensionsstellung bei der Einstellung des Slope berücksichtigt werden.

Bevor auf weitere Einzelheiten der Funktion des chirurgischen Instruments 1 und dessen Verwendung eingegangen werden, wird zunächst der weitere Aufbau des chirurgischen Instruments 1 im Detail erläutert.

Die erste Befestigungseinrichtung 100 weist bei der gezeigten Ausführungsform ein Steckelement 101 auf, welches zum lösbaren Zusammenstecken mit einer Referenzgeometrie des Femurschnittblocks 600 eingerichtet ist. Der Femurschnittblock 600 dient einer Anbringung eines distalen Femurschnitts und kann auch als Femursägeblock oder distal femur cutting jig bezeichnet werden. Die Gestaltung und Funktionsweise des Femurschnittblocks 600 sind dem Fachmann grundsätzlich bekannt. Der Femurschnittblock 600 weist einen Führungsschlitz 601 auf, der vorliegend als Referenzgeometrie dient. Der Führungsschlitz 601 ist zur Aufnahme und Führung eines medizinischen Sägeblatts zur distalen Resektion des Femurs F eingerichtet und wird im Rahmen des Kinematic Alignment-Ansatzes üblicherweise parallel zu einer distalen Kondylenlinie des Femurs F ausgerichtet. Das Steckelement 101 kann zur Befestigung des chirurgischen Instruments 1 an dem Femurschnittblock 600 in dessen Führungsschlitz 601 eingesteckt werden. Ein solcher Zustand ist anhand der Fig. 1 bis 5 gezeigt. Das Steckelement 101 ist zu diesem Zweck maßlich auf den Führungsschlitz 601 abgestimmt. In eingestecktem Zustand ist das Steckelement 101 mediolateral, proximodistal sowie posterior formschlüssig in dem Führungsschlitz 601 gehalten. Zum Lösen der Befestigung kann das Steckelement 101 anterior aus dem Führungsschlitz 601 herausgezogen werden.

Die erste Linearführung 400 ist bei der gezeigten Ausführungsform eine Zylinderführung Z und weist wenigstens ein entlang der ersten Führungsbahn längserstrecktes Zylinderelement 401 und eine koaxial zu derselben längserstreckte Zylinderaufnahme 402 auf. Das Zylinderelement 401 ist entlang der ersten Führungsbahn L1 gleitbeweglich in der Zylinderaufnahme 402 aufgenommen.

Vorliegend ist das Zylinderelement 401 fest und/oder (relativ) unbeweglich mit der ersten Befestigungseinrichtung 100, genauer: dessen Steckelement 101, verbunden. Die Zylinderaufnahme 402 ist fest und/oder (relativ) unbeweglich mit der Kreisbogenführung 300 verbunden. Bei einer nicht in den Figuren gezeigten Ausführungsform ist eine hierzu umgekehrte Anordnung vorgesehen.

Das Zylinderelement 401 und die Zylinderaufnahme 402 weisen jeweils einen kreiszylindrischen Querschnitt auf. Um einer ungewollten Rotation der Zylinderführung Z um ihre Längsachse entgegenzuwirken, ist vorliegend ein weiteres Zylinderelement 401' und eine weitere Zylinderaufnahme 402` vorgesehen. Das weitere Zylinderelement 401' und die weitere Zylinderaufnahme 402` sind medial von dem Zylinderelement 401 und der Zylinderaufnahme 402 beabstandet angeordnet. Im Übrigen sind deren Funktionsweise und Gestaltung identisch. Weitere Erläuterungen zu dem weiteren Zylinderelement 401' und der weiteren Zylinderaufnahme 402` sind daher entbehrlich. Bei einer nicht in den Figuren gezeigten Ausführungsform weist die Zylinderführung nicht rotationsunsymmetrische Querschnitte auf, um einer ungewollten Rotation um die erste Führungsbahn entgegenzuwirken.

Die Kreisbogenführung 300 weist bei der gezeigten Ausführungsform eine gebogene Führungsstange 301 und eine komplementär gebogene Führungsaufnahme 302 auf. Die Führungsstange 301 ist entlang der Kreisführungsbahn C gleitbeweglich in der Führungsaufnahme 302 aufgenommen.

Bei der gezeigten Ausführungsform ist die Führungsaufnahme 302 in Bezug auf die Kreisführungsbahn C feststehend und/oder unbeweglich mit der ersten Befestigungseinrichtung 100 verbunden. Dementgegen ist die Führungsstange 301 in Bezug auf die Kreisführungsbahn C relativbeweglich zu der ersten Befestigungseinrichtung 100. Sowohl die Führungsstange 301 als auch die Führungsaufnahme 302 sind in Bezug auf die erste Führungsbahn L1 relativbeweglich zu der ersten Befestigungseinrichtung 100.

Bei der gezeigten Ausführungsform weist die Führungsaufnahme 302 einen U-förmigen Querschnitt mit mediolateral gegenüberliegenden Schenkeln 3021, 3022 auf. Die Schenkel 3021, 3022 können auch als lateraler Schenkel 3021 und medialer Schenkel 3022 bezeichnet werden. Die Führungsstange 301 ist mediolateral formschlüssig zwischen den beiden Schenkeln 3021, 3022 gehalten. Die Führungsaufnahmen 402, 402` der Zylinderführung Z sind bei der gezeigten Ausführungsform fest und/oder unbeweglich mit der Führungsaufnahme 302 verbunden. Vorliegend bilden die Führungsaufnahme 302 und die beiden Zylinderaufnahmen 402, 402` ein gemeinsames, einstückiges Bauteil.

Die Führungsstange 301 ist zwischen einem proximalen Ende und einem distalen Ende (jeweils ohne Bezugszeichen) gebogen längserstreckt. Das proximale Ende ist gleitbeweglich in der Führungsaufnahme 302 gehalten. Das distale Ende ist mittels der zweiten Linearführung 500 mit der zweiten Befestigungseinrichtung 200 entlang der zweiten Führungsbahn L2 relativbeweglich verbunden.

Vorliegend weist die Kreisbogenführung 300 zudem eine Fixiereinrichtung 303 auf, welche zur Fixierung der Beweglichkeit der Kreisbogenführung 300 eingerichtet ist. Mittels der Fixiereinrichtung 300 kann die Relativbeweglichkeit zwischen der Führungsstange 301 und der Führungsaufnahme 302 wahlweise fixiert und gelöst werden. Die Fixiereinrichtung 303 wirkt folglich einer ungewollten Relativbewegung entlang der Kreisführungsbahn C entgegen. Die Fixiereinrichtung 303 bewirkt eine lösbare kraft- und/oder formschlüssige Verbindung zwischen der Führungsstange 301 und der Führungsaufnahme 302. Beispielsweise kann die Fixiereinrichtung 303 ein Verspannen der Schenkel 3021, 3022 gegenüber der Führungsstange 301 bewirken. Zu diesem Zweck kann die Fixiereinrichtung 302 einen Schraub-, Klemm-, Kast- oder sonstigen Mechanismus aufweisen. Weitere Details zur Funktionsweise und zum Aufbau der Fixiereinrichtung 303 sind für die vorliegende Erfindung nicht wesentlich. Eine detailliertere Erläuterung der Fixiereinrichtung 303 wird daher als entbehrlich erachtet.

Weiter weist die Kreisbogenführung 300 vorliegend eine erste Anzeigeeinrichtung A1 auf. Die erste Anzeigeeinrichtung A1 ist bei der gezeigten Ausführungsform eine Skale mit nicht näher bezeichneten Teilstrichen, welche vorliegend an der Führungsstange 301 angebracht sind. Eine nicht näher bezeichnete distale Vorderkante der Führungsaufnahme 302 kann als Ablesemarke dienen. Bei einer Relativbewegung zwischen der Führungsstange 301 und der Führungsaufnahme 302 wandert die Ablesemarke entlang der Teilstriche. Die erste Anzeigeeinrichtung A1 zeigt folglich eine relative Position zwischen der Führungsstange 301 und der Führungsaufnahme 302 in Bezug auf die Kreisführungsbahn C an. In der Verwendung des chirurgischen Instruments 1 zeigt die erste Anzeigeeinrichtung A1 folglich eine erste Winkelstellung α1 der zweiten Befestigungseinrichtung 200 - und damit auch des Tibiaschnittblocks 700 - in Bezug auf die kreisbogenförmige Führungsbahn C an. Mit anderen Worten dient die erste Anzeigeeinrichtung A1 einer Anzeige der sagittalen Neigung, d.h. des Slope, des Tibiaschnittblocks 700.

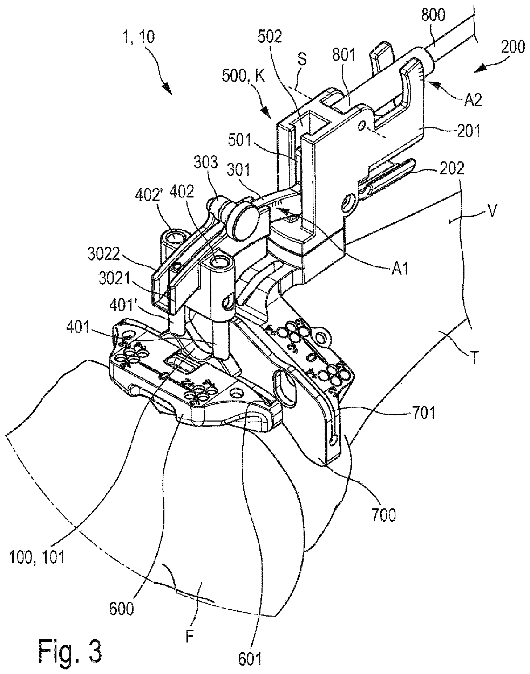

Die zweite Linearführung 500 ist bei der gezeigten Ausführungsform eine Kulissenführung K. Die Kulissenführung K weist einen Kulissenstein 501 (siehe insbesondere Fig. 3) und einen Kulissenschlitz 502 auf. Der Kulissenschlitz 502 ist entlang der zweiten Führungsbahn L2 längserstreckt. Der Kulissenstein 501 ist entlang der zweiten Führungsbahn L2 gleitbeweglich in dem Kulissenschlitz 502 gehalten.

Bei der gezeigten Ausführungsform ist der Kulissenstein 501 fest und/oder (relativ) unbeweglich mit der Kreisbogenführung 300 verbunden. Der Kulissenschlitz 502 ist fest und/oder (relativ) unbeweglich mit der zweiten Befestigungseinrichtung 200 verbunden. Im Speziellen ist der Kulissenstein 502 fest mit dem distalen Ende (ohne Bezugszeichen) der Führungsstange 301 verbunden. Der Kulissenschlitz 502 ist einends, an seinem anterioren Ende (ohne Bezugszeichen) offen. Der Kulissenstein 501 kann durch das offene Ende in den Kulissenschlitz 502 eingeführt und aus demselben herausgenommen werden. Dies erlaubt insbesondere ein einfaches Auseinandernehmen und Wiederzusammenfügen des chirurgischen Instruments 1, beispielsweise zur Reinigung, Sterilisierung oder dergleichen.

Die zweite Befestigungseinrichtung 200 weist bei der gezeigten Ausführungsform einen Grundkörper 201 auf. Der Kulissenschlitz 502 ist vorliegend an dem Grundkörper 201 ausgebildet. Zur eigentlichen Befestigung des Tibiaschnittblocks 700 an der zweiten Befestigungseinrichtung 200 weist dieselbe ein Befestigungselement 202 auf. Das Befestigungselement 202 wirkt lösbar kraft- und/oder formschlüssig mit einem hierfür vorgesehenen Abschnitt des Tibiaschnittblocks 700 zusammen. Vorliegend ist das Befestigungselement 202 zwischen einer Befestigungsstellung und einer Freigabestellung relativ zu dem Grundkörper 201 an demselben gelagert. In der Befestigungsstellung ist der Tibiaschnittblock 700 mittels des Befestigungselements 202 lösbar mit dem Grundkörper 201 verbunden. In der Freigabestellung ist der Tibiaschnittblock 700 relativ zu dem Grundkörper 201 freigegeben und umgekehrt.

Bei der gezeigten Ausführungsform weist das chirurgische Instrument zudem einen Ausrichtstab 800 auf. Der Ausrichtstab 800 ist zum Ausrichten an einer anterioren Kante V der Tibia T eingerichtet. Die anteriore Kante V kann auch als Tibiavorderkante bezeichnet werden. Der Ausrichtstab 800 ist einends relativ zu der zweiten Befestigungseinrichtung 200 um eine orthogonal zu der Führungsebene E orientierte Schwenkachse S schwenkbeweglich an der zweiten Befestigungseinrichtung 200 gelagert. Bei der gezeigten Ausführungsform ist der Ausrichtstab 800 an dem Grundkörper 201 gelagert. Dabei ist der Ausrichtstab 800 zwischen einem proximalen Ende 801 und einem (nicht im Detail gezeigten) distalen Ende 802 längserstreckt. Die schwenkbewegliche Lagerung erlaubt eine Ausrichtung des Ausrichtstabs 800 in der Führungsebene E und/oder parallel zu derselben. Hierdurch kann der Ausrichtstab 800 insbesondere zu einer Kontrolle der Position des Tibiaschnittblocks 700 und/oder zur Kontrolle der Extensionsstellung der Tibia T parallel zu der Tibiavorderkante V ausgerichtet werden. Die spezielle Funktion des Ausrichtstabs 800 wird nachfolgend noch näher erläutert.

Dem Ausrichtstab 800 ist bei der gezeigten Ausführungsform eine zweite Anzeigeeinrichtung A2 zugeordnet. Die zweite Anzeigeeinrichtung A2 ist zum Anzeigen einer zweiten Winkelstellung α2 des Ausrichtstabs 800 in Bezug auf die Schwenkachse S eingerichtet. Vorliegend ist die zweite Anzeigeeinrichtung A2 eine Skale mit mehreren Teilstrichen (ohne Bezugszeichen). Die Teilstriche sind vorliegend an der zweiten Befestigungseinrichtung 200, genauer: deren Grundkörper 201, angebracht. Zudem ist eine nicht näher gezeigte Ablesemarke vorhanden, wobei diese beispielsweise auch durch den Ausrichtstab 800 selbst gebildet sein kann.

Nachfolgend wird eine exemplarische Verwendung des chirurgischen Instruments 1 zur Positionierung des Tibiaschnittblocks 700 erläutert.

Die Positionierung erfolgt ausgehend von einer Konfiguration, in welcher der distale Femurschnittblock 600 bereits auf eine dem Fachmann bekannte Weise an dem distalen Femur F fixiert ist. D.h. der bereits fixierte Femurschnittblock 600 dient als Referenz. Der Führungsschlitz 601 ist hierbei mediolateral sowie anteroposterior erstreckt. Eine rotatorische Ausrichtung des Führungsschlitzes 601 in Bezug auf eine anteroposteriore Achse gibt dabei eine Varus/Valgus-Ausrichtung des anzubringenden distalen Femurschnitts vor. Vorliegend wird davon ausgegangen, dass die Varus/Valgus-Ausrichtung 0° beträgt. Der Führungsschlitz 601 ist folglich parallel zu einer distalen Kondylenlinie des distalen Femurs F orientiert. Mittels des chirurgischen Instruments 1 kann insbesondere eine femurseitig bereits vorgenommeine Varus/Valgus-Ausrichtung auf den Tibiaschnittblock 700, genauer: dessen Führungsschlitz 701, transferiert werden.

Hierfür wird zunächst der Tibiaschnittblock 700 auf die vorbeschriebene Weise an der zweiten Befestigungseinrichtung 200 lösbar befestigt. Das chirurgische Instrument 1 wird sodann gemeinsam mit dem an demselben befestigten Tibiaschnittblock 700 femurseitig referenziert. Zu diesem Zweck wird die erste Befestigungseinrichtung 100 lösbar an dem bereits femurseitig fixierten Femurschnittblock 600 befestigt. Dies auf die vorbeschriebene Weise, d.h. das Steckelement 101 wird posterior in den Führungsschlitz 601 eingesteckt. Hiernach ergibt sich die anhand der Fig. 1 bis 4 gezeigte Konfiguration.

In dieser (exemplarischen) Konfiguration liegt eine exakte Extension vor, in welcher der Femur F und die Tibia T einen Winkel von 180° einschließen. Ausgehend von einer solchen (vollständigen) Extension erfolgt die Einstellung der sagittalen Neigung des Führungsschlitzes 701 wie folgt:
Zunächst wird der Mittelpunkt M der Kreisbogenführung 300 an die Anatomie des Patienten angepasst positioniert. Diese Positionierung erfolgt mittels der ersten Linearführung 400. Bei einer Verlagerung der Kreisbogenführung 300 entlang der ersten Führungsbahn L1 in posteriorer Richtung bewegt der Mittelpunkt M sich posterior. Umgekehrt bewirkt eine anterior gerichtete Relativbewegung der Kreisbogenführung 300 gegenüber der ersten Befestigungseinrichtung 100 - und damit dem Femurschnittblock 600 - eine anteriore Verlagerung des Mittelpunkts M. Es versteht sich, dass das Steckelement 101 hierbei stets in den Führungsschlitz 601 eingesteckt bleibt. Der Mittelpunkt M wird im Idealfall mittig auf eine nicht näher eingezeichnete (mechanische) Tibiaachse positioniert. Mit einer solchen Positionierung des Mittelpunkts M wird vermieden, dass eine Einstellung der Neigung des Tibiaschnittblocks 700 gleichzeitig mit einer ungewollten Veränderung einer proximodistalen Position in Bezug auf die Tibia einhergeht (Tibiaschnitthöhe).

Nach erfolgter Positionierung des Mittelpunkts M über die vorbeschriebene Einstellung der ersten Linearführung 400 wird der Tibiaschnittblock 700 anterior an der Tibia T anliegend positioniert. Dabei gewährleistet die zweite Linearführung 500 eine stets anforderungsgerechte Anlage. Der Tibiaschnittblock 700 kann mittels einer Verlagerung der zweiten Befestigungseinrichtung 200 entlang der zweiten Führungsachse L2 in Anpassung an vorherrschende Abmessungen der Tibia T angehoben oder abgesenkt werden.

In einem weiteren Schritt erfolgt die eigentliche Einstellung der sagittalen Neigung des Führungsschlitzes 701, d.h. des Slope. In den Fig. 1 bis 4 beträgt der Slope 0°, so dass der Führungsschlitz 701 des Tibiaschnittblocks 700 planparallel zu dem Führungsschlitz 601 des Femurschnittblocks 600 ausgerichtet ist. Die erste Winkelstellung α1 (Fig. 1) repräsentiert hierbei den besagten Slope. Der Slope kann über die Kreisbogenführung 300 eingestellt werden. Der eingestellte Wert ist mittels der ersten Anzeigeeinrichtung A1 ablesbar. Durch die zuvor erfolgte Positionierung des Mittelpunkts M auf der Tibiaachse wird die Tibiaschnitthöhe hierbei nicht verändert.

Der Ausrichtstab 800 dient hierbei zum einen einer Kontrolle der vollständigen Extension. Bei einer vollständigen Extension und parallelen Ausrichtung des Ausrichtstabs 800 zu der Tibiavorderkante V beträgt die zweite Winkelstellung α2 vorliegend 0°.

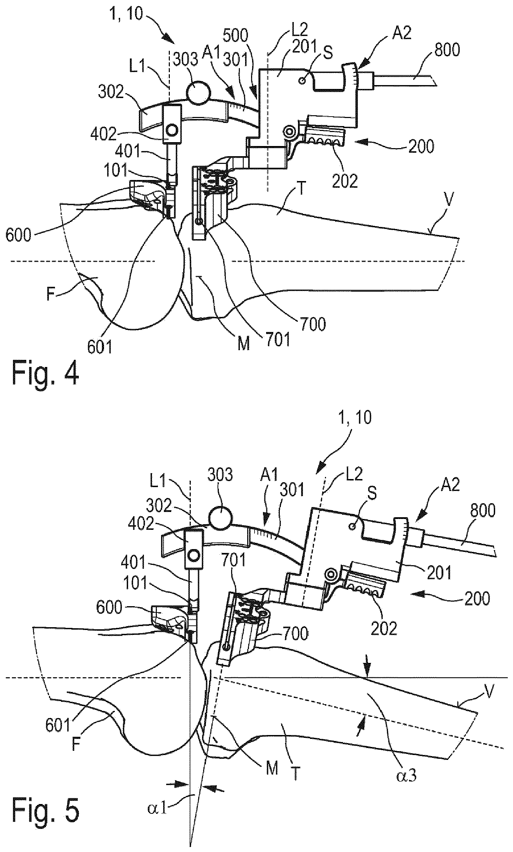

Der Ausrichtstab 800 ermöglicht zusammen mit den beiden Anzeigeeinrichtungen A1, A2 zudem eine Flexion/Extensions-Korrektur. Sofern keine exakte Extension vorliegen sollte, kann dies mittels des Ausrichtstabs 800 und der zweiten Anzeigeeinrichtung A2 ermittelt und über eine entsprechende Einstellung des Slope an der zweiten Anzeigeeinrichtung A1 ausgeglichen werden. Zur Erläuterung wird nachfolgend auf die in Fig. 5 dargestellte exemplarische Situation eingegangen.

In der anhand Fig. 5 gezeigten Situation liegt keine exakte Extension vor. Stattdessen ist die Tibia T um einen Winkel α3 relativ zu dem Femur F angewinkelt. Das chirurgische Instrument 1 erlaubt eine Berücksichtigung des Winkels α3 bei der Einstellung des Slope. Hierfür wird zunächst das chirurgische Instrument 1 mitsamt des Tibiaschnittblocks 700 auf die vorbeschriebene Weise mit dem bereits fixierten Femurschnittblock 600 verbunden. Die Kreisbogenführung 300 wird hiernach so eingestellt, dass die Anzeigeeinrichtung A1 eine erste Winkelstellung α1 anzeigt, die einen Slope von 0° repräsentiert. Der tatsächliche Slope beträgt hierbei - aufgrund der vorliegenden Flexion - nicht 0°. Hiernach wird der Winkel α3 ermittelt, in dem der Ausrichtstab 800 parallel zu der Tibiavorderkante V ausgerichtet wird. Der Winkel α3 kann an der zweiten Anzeigeeinrichtung A2 abgelesen werden. Hiernach wird der entsprechende Winkel an der Kreisbogenführung 300 eingestellt. Dies bewirkt, dass der Führungsschlitz 701 - trotz Abweichung von der exakten Extensionsstellung - orthogonal zu der Tibiavorderkante V und somit in einem Slope von 0° eingestellt ist. Ausgehend von dieser (Grund-)Einstellung kann eine weitere Anpassung des Slope über eine entsprechende Verlagerung der Kreisbogenführung 300 vorgenommen werden.

## Patentansprüche

1. Chirurgisches Instrument (1) zur Verwendung bei einer Kniegelenkersatzoperation, aufweisend
eine erste Befestigungseinrichtung (100), welche zur lösbaren Befestigung an einer an einem distalen Femur (F) angebrachten Referenzkomponente (600) eingerichtet ist,
eine zweite Befestigungseinrichtung (200), welche distal von der ersten Befestigungseinrichtung (100) beabstandet und zur lösbaren Befestigung an einem Tibiaschnittblock (700) zur Schnittführung an einer proximalen Tibia (T) eingerichtet ist,
eine Kreisbogenführung (300), welche einends mit der ersten Befestigungseinrichtung (100) und andernends mit der zweiten Befestigungseinrichtung (200) verbunden ist und mittels derer die zweite Befestigungseinrichtung (200) relativ zu der ersten Befestigungseinrichtung (100) in einer sagittalen Führungsebene (E) entlang einer kreisbogenförmig längserstreckten Führungsbahn (C) geführt linearbeweglich ist,
eine erste Linearführung (400), mittels derer die Kreisbogenführung (300) einends mit der ersten Befestigungseinrichtung (100) verbunden und relativ zu derselben in der Führungsebene (E) entlang einer anteroposterior längserstreckten ersten Führungsbahn (L1) geführt linearbeweglich ist,
und eine zweite Linearführung (500), mittels derer die zweite Befestigungseinrichtung (200) andernends mit der Kreisbogenführung (300) verbunden und relativ zu derselben in der Führungsebene (E) entlang einer anteroposterior längserstreckten zweiten Führungsbahn (L2) geführt linearbeweglich ist.

2. Chirurgisches Instrument (1) nach Anspruch 1, **dadurch gekennzeichnet, dass** ein Ausrichtstab (800) vorhanden und zur Ausrichtung an einer anterioren Kante (V) der Tibia (T) eingerichtet ist, wobei der Ausrichtstab (800) einends relativ zu der zweiten Befestigungseinrichtung (200) um eine orthogonal zu der Führungsebene (E) orientierte Schwenkachse (S) schwenkbeweglich an der zweiten Befestigungseinrichtung (200) gelagert ist.

3. Chirurgisches Instrument (1) nach Anspruch 1 oder 2, **dadurch gekennzeichnet, dass** eine erste Anzeigeeinrichtung (A1) vorhanden und zum Anzeigen einer ersten Winkelstellung (α1) der zweiten Befestigungseinrichtung (200) in Bezug auf die kreisbogenförmige Führungsbahn (C) eingerichtet ist, und/oder dass eine zweite Anzeigeeinrichtung (A2) vorhanden und zum Anzeigen einer zweiten Winkelstellung (α2) des Ausrichtstabs (800) in Bezug auf die zweite Schwenkachse (S) eingerichtet ist.

4. Chirurgisches Instrument (1) nach einem der vorhergehenden Ansprüche, **dadurch gekennzeichnet, dass** die Kreisbogenführung (300) wenigstens eine gebogene Führungsstange (301) und eine Führungsaufnahme (302) aufweist, in welcher die Führungsstange (301) gleitbeweglich geführt aufgenommen ist, wobei die Führungsaufnahme (302) mit der ersten Linearführung (400) und die Führungsstange (301) mit der zweiten Linearführung (500) verbunden ist oder umgekehrt.

5. Chirurgisches Instrument (1) nach Anspruch 4, **dadurch gekennzeichnet, dass** die Führungsaufnahme (302) mit der ersten Linearführung (400) verbunden ist und ein Führungselement, insbesondere ein Zylinderelement (401) oder eine Zylinderaufnahme (402), derselben aufweist, und/oder dass die Führungsstange (301) mit der zweiten Linearführung (500) verbunden ist und ein Führungselement, insbesondere einen Kulissenstein (501) oder einen Kulissenschlitz (502), derselben aufweist.

6. Chirurgisches Instrument (1) nach einem der vorhergehenden Ansprüche, **dadurch gekennzeichnet, dass** die erste Linearführung (400) eine Zylinderführung (Z) ist und wenigstens ein anteroposterior längserstrecktes Zylinderelement (401) und eine Zylinderaufnahme (402) aufweist, in welcher das Zylinderelement (401) gleitbeweglich geführt aufgenommen ist.

7. Chirurgisches Instrument (1) nach Anspruch 6, **dadurch gekennzeichnet, dass** das Zylinderelement (401) unbeweglich mit der ersten Befestigungseinrichtung (100) verbunden ist, und dass die Zylinderaufnahme (402) unbeweglich mit der Kreisbogenführung (300), vorzugsweise deren Führungsaufnahme (302), verbunden ist.

8. Chirurgisches Instrument (1) nach einem der vorhergehenden Ansprüche, **dadurch gekennzeichnet, dass** die zweite Linearführung (500) eine Kulissenführung (K) ist und einen Kulissenstein (501) und einen anteroposterior längserstreckten Kulissenschlitz (502) aufweist, in welchem der Kulissenstein (501) gleitbeweglich geführt aufgenommen ist.

9. Chirurgisches Instrument (1) nach Anspruch 8, **dadurch gekennzeichnet, dass** der Kulissenstein (501) unbeweglich mit der Kreisbogenführung (300), vorzugsweise deren Führungsstange (301), verbunden ist, und dass der Kulissenschlitz (502) an der zweiten Befestigungseinrichtung (200) ausgebildet ist.

10. Chirurgisches Instrument (1) nach einem der vorhergehenden Ansprüche, **dadurch gekennzeichnet, dass** die erste Befestigungseinrichtung (100) wenigstens ein Steckelement (101) aufweist, welches zum lösbaren Zusammenstecken mit einer komplementären Referenzgeometrie (601) der Referenzkomponente (600) eingerichtet ist.

11. Chirurgisches Instrument (1) nach einem der vorhergehenden Ansprüche, **dadurch gekennzeichnet, dass** die zweite Befestigungseinrichtung (200) einen Grundkörper (201) aufweist, an welchem ein Befestigungselement (202), welches zur lösbaren Befestigung an einem hierfür vorgesehenen Befestigungsabschnitt des Tibiaschnittblocks (700) eingerichtet ist, ein Führungselement der zweiten Linearführung (500), vorzugsweise deren Kulissenschlitz (502), und der Ausrichtstab (800) angeordnet sind.

12. Chirurgisches Instrument (1) nach einem der vorhergehenden Ansprüche, **dadurch gekennzeichnet, dass** eine Fixiereinrichtung (303) vorhanden und zur Fixierung der Beweglichkeit der Kreisbogenführung (300) eingerichtet ist.

13. Chirurgisches Instrumentensystem (10) mit einem chirurgischen Instrument (1) nach einem der vorhergehenden Ansprüche, einer Referenzkomponente (600), die lösbar an der ersten Befestigungseinrichtung (100) des chirurgischen Instruments (1) befestigt ist, und mit einem Tibiaschnittblock (700), der lösbar an der zweiten Befestigungseinrichtung (200) des chirurgischen Instruments (1) befestigt ist.

## Claims

1. Surgical instrument (1) for use in a knee joint replacement operation, having
a first fastening device (100) which is configured for releasably fastening to a reference component (600) attached to a distal femur (F),
a second fastening device (200) which is spaced apart distally from the first fastening device (100) and is configured for releasably fastening to a tibial cutting block (700) for cutting guidance on a proximal tibia (T),
a circular arc guide (300) which is connected at one end to the first fastening device (100) and at the other end to the second fastening device (200) and by means of which the second fastening device (200) is linearly movable relative to the first fastening device (100) guided in a sagittal guide plane (E) along a guide path (C) which is elongate in the manner of a circular arc,
a first linear guide (400), by means of which the circular arc guide (300) is connected at one end to the first fastening device (100) and is linearly movable relative thereto guided in the guide plane (E) along a first guide path (L1) which is elongate anteroposteriorly,
and a second linear guide (500), by means of which the second fastening device (200) is connected at the other end to the circular arc guide (300) and is linearly movable relative thereto guided in the guide plane (E) along a second guide path (L2) which is elongate anteroposteriorly.

2. Surgical instrument (1) according to Claim 1, **characterized in that** an alignment rod (800) is present and is configured for aligning on an anterior edge (V) of the tibia (T), wherein the alignment rod (800) is mounted at one end on the second fastening device (200) so as to be pivotable relative to the second fastening device (200) about a pivot axis (S) oriented orthogonally to the guide plane (E).

3. Surgical instrument (1) according to Claim 1 or 2, **characterized in that** a first indicating device (A1) is present and is configured for indicating a first angular position (α1) of the second fastening device (200) with respect to the circular-arc-shaped guide path (C), and/or in that a second indicating device (A2) is present and is configured for indicating a second angular position (α2) of the alignment rod (800) with respect to the second pivot axis (S).

4. Surgical instrument (1) according to one of the preceding claims, **characterized in that** the circular arc guide (300) has at least one curved guide rod (301) and a guide receptacle (302) in which the guide rod (301) is accommodated in a slidably guided manner, wherein the guide receptacle (302) is connected to the first linear guide (400) and the guide rod (301) to the second linear guide (500), or vice versa.

5. Surgical instrument (1) according to Claim 4, **characterized in that** the guide receptacle (302) is connected to the first linear guide (400) and has a guide element, in particular a cylinder element (401) or a cylinder receptacle (402), of same, and/or **in that** the guide rod (301) is connected to the second linear guide (500) and has a guide element, in particular a slotted guide block (501) or a slotted guide slot (502) , of same.

6. Surgical instrument (1) according to one of the preceding claims, **characterized in that** the first linear guide (400) is a cylinder guide (Z) and has at least one cylinder element (401), which is elongate anteroposteriorly, and a cylinder receptacle (402) in which the cylinder element (401) is accommodated in a slidably guided manner.

7. Surgical instrument (1) according to Claim 6, **characterized in that** the cylinder element (401) is connected immovably to the first fastening device (100), and **in that** the cylinder receptacle (402) is connected immovably to the circular arc guide (300), preferably to the guide receptacle (302) thereof.

8. Surgical instrument (1) according to one of the preceding claims, **characterized in that** the second linear guide (500) is a slotted guide mechanism (K) and has a slotted guide block (501) and a slotted guide slot (502) which is elongate anteroposteriorly and in which the slotted guide block (501) is accommodated in a slidably guided manner.

9. Surgical instrument (1) according to Claim 8, **characterized in that** the slotted guide block (501) is connected immovably to the circular arc guide (300), preferably to the guide rod (301) thereof, and **in that** the slotted guide slot (502) is formed on the second fastening device (200).

10. Surgical instrument (1) according to one of the preceding claims, **characterized in that** the first fastening device (100) has at least one plug-in element (101) which is configured for releasably plugging together with a complementary reference geometry (601) of the reference component (600).

11. Surgical instrument (1) according to one of the preceding claims, **characterized in that** the second fastening device (200) has a main body (201) on which a fastening element (202), which is configured for releasably fastening to a fastening portion, provided for this purpose, of the tibial cutting block (700), a guide element of the second linear guide (500), preferably the slotted guide slot (502) thereof, and the alignment rod (800) are arranged.

12. Surgical instrument (1) according to one of the preceding claims, **characterized in that** a fixing device (303) is present and is configured for fixing the movability of the circular arc guide (300).

13. Surgical instrument system (10) with a surgical instrument (1) according to one of the preceding claims, a reference component (600), which is fastened releasably to the first fastening device (100) of the surgical instrument (1), and with a tibial cutting block (700), which is fastened releasably to the second fastening device (200) of the surgical instrument (1).

## Revendications

1. Instrument chirurgical (1) destiné à être utilisé dans la chirurgie d'arthroplastie du genou, ledit instrument comprenant
un premier dispositif de fixation (100), qui est conçu pour être fixé de manière amovible à un composant de référence (600) monté sur un fémur distal (F),
un deuxième dispositif de fixation (200), qui est espacé distalement du premier dispositif de fixation (100) et qui est conçu pour être fixé de manière amovible à un bloc de coupe de tibia (700) afin de réaliser une incision sur un tibia proximal (T),
un guide en arc de cercle (300), qui est relié à une extrémité au premier dispositif de fixation (100) et à l'autre extrémité au deuxième dispositif de fixation (200) et au moyen duquel le deuxième dispositif de fixation (200) est mobile linéairement par rapport au premier dispositif de fixation (100) dans un plan de guidage sagittal (E) en étant guidé le long d'une piste de guidage (C) s'étendant longitudinalement en forme d'arc de cercle,
un premier guide linéaire (400), au moyen duquel le guide en arc de cercle (300) est relié à une extrémité au premier dispositif de fixation (100) et qui est mobile linéairement par rapport à celui-ci dans le plan de guidage (E) en étant guidé le long d'une première piste de guidage (L1) s'étendant longitudinalement suivant une direction antéropostérieure,
et un deuxième guide linéaire (500), au moyen duquel le deuxième dispositif de fixation (200) est relié à l'autre extrémité au guide en arc de cercle (300) et qui est mobile linéairement par rapport à celui-ci dans le plan de guidage (E) en étant guidé le long d'une deuxième piste de guidage (L2) s'étendant longitudinalement suivant une direction antéropostérieure.

2. Instrument chirurgical (1) selon la revendication 1, **caractérisé en ce qu'**une tige d'alignement (800) est présente et est conçue pour effectuer un alignement sur un bord antérieur (V) du tibia (T), la tige d'alignement (800) étant montée sur le deuxième dispositif de fixation (200) de manière à pouvoir pivoter à une extrémité par rapport au deuxième dispositif de fixation (200) sur un axe de pivotement (S) orienté orthogonalement au plan de guidage (E).

3. Instrument chirurgical (1) selon la revendication 1 ou 2, **caractérisé en ce qu'**un premier dispositif d'affichage (A1) est présent et est conçu pour afficher une première position angulaire (α1) du deuxième dispositif de fixation (200) par rapport à la piste de guidage en forme d'arc de cercle (C), et/ou **en ce qu'**un deuxième dispositif d'affichage (A2) est présent et est conçu pour afficher une deuxième position angulaire (α2) de la tige d'alignement (800) par rapport au deuxième axe de pivotement (S).

4. Instrument chirurgical (1) selon l'une des revendications précédentes, **caractérisé en ce que** le guide en arc de cercle (300) comporte au moins une tige de guidage incurvée (301) et un logement de guidage (302) dans lequel la tige de guidage (301) est logée en étant guidée de manière coulissante,
le logement de guidage (302) étant relié au premier guide linéaire (400) et la tige de guidage (301) étant reliée au deuxième guide linéaire (500) ou vice versa.

5. Instrument chirurgical (1) selon la revendication 4, **caractérisé en ce que** le logement de guidage (302) est relié au premier guide linéaire (400) et comporte un élément de guidage, notamment un élément cylindrique (401) ou un logement cylindrique (402), de celui-ci, et/ou **en ce que** la tige de guidage (301) est reliée au deuxième guide linéaire (500) et comporte un élément de guidage, notamment un coulisseau (501) ou une fente de coulisse (502), de celui-ci.

6. Instrument chirurgical (1) selon l'une des revendications précédentes, **caractérisé en ce que** le premier guide linéaire (400) est un guide cylindrique (Z) et comporte au moins un élément cylindrique (401) s'étendant longitudinalement suivant une direction antéropostérieure et un logement cylindrique (402) dans lequel l'élément cylindrique (401) est logé en étant guidé de manière coulissante.

7. Instrument chirurgical (1) selon la revendication 6, **caractérisé en ce que** l'élément cylindrique (401) est relié de manière immobile au premier dispositif de fixation (100), et **en ce que** le logement cylindrique (402) est relié de manière immobile au guide en arc de cercle (300), de préférence son logement de guidage (302) .

8. Instrument chirurgical (1) selon l'une des revendications précédentes, **caractérisé en ce que** le deuxième guide linéaire (500) est un guide de coulisse (K) et comporte un coulisseau (501) et une fente de coulisse qui s'étend longitudinalement suivant une direction antéropostérieure (502) et dans laquelle le coulisseau (501) est logé en étant guidé de manière coulissante.

9. Instrument chirurgical (1) selon la revendication 8, **caractérisé en ce que** le coulisseau (501) est relié de manière immobile au guide en arc de cercle (300), de préférence à sa tige de guidage (301), et **en ce que** la fente de coulisse (502) est formée sur le deuxième dispositif de fixation (200).

10. Instrument chirurgical (1) selon l'une des revendications précédentes, **caractérisé en ce que** le premier dispositif de fixation (100) comporte au moins un élément enfichable (101) qui est conçu pour s'enficher de manière amovible avec une géométrie de référence complémentaire (601) du composant de référence (600).

11. Instrument chirurgical (1) selon l'une des revendications précédentes, **caractérisé en ce que** le deuxième dispositif de fixation (200) comporte un corps de base (201) sur lequel sont disposés un élément de fixation (202) qui est conçu pour être fixé de manière amovible à une portion de fixation, prévue pour cela, du bloc de coupe de tibia (700), un élément de guidage du deuxième guide linéaire (500), de préférence sa fente de coulisse (502), et la tige d'alignement (800).

12. Instrument chirurgical (1) selon l'une des revendications précédentes, **caractérisé en ce qu'**un dispositif d'immobilisation (303) est présent et est conçu pour immobiliser la mobilité du guide en arc de cercle (300).

13. Système d'instrument chirurgical (10) comprenant un instrument chirurgical (1) selon l'une des revendications précédentes, un composant de référence (600) qui est fixé de manière amovible au premier dispositif de fixation (100) de l'instrument chirurgical (1), et un bloc de coupe de tibia (700), qui est fixé de manière amovible au deuxième dispositif de fixation (200) de l'instrument chirurgical (1).
